(19) Europäisches Patentamt
European Patent Office
Office européen des brevets

(11) **EP 1 216 691 A1**

(12) **EUROPEAN PATENT APPLICATION**

(43) Date of publication:
**26.06.2002 Bulletin 2002/26**

(21) Application number: **00128472.8**

(22) Date of filing: **23.12.2000**

(51) Int Cl.[7]: **A61K 7/46**, A61K 7/32, A61K 7/48, C11D 3/00, C11D 3/28, C11D 3/32

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Givaudan SA**
**1214 Vernier-Genève (CH)**

(72) Inventor: **Natsch, Andreas**
**8640 Rapperswil (CH)**

(74) Representative: **Patentanwälte**
**Schaad, Balass, Menzl & Partner AG**
**Dufourstrasse 101**
**Postfach**
**8034 Zürich (CH)**

(54) **Antibacterial composition comprising a synergistic mixture of a fragrance aldehyde and a preservative**

(57) The present invention relates to an antibacterial composition comprising a synergistic mixture of p-methylphenylacetaldehyde or phenylacetaldehyde or a mixture thereof and a preservative selected from of diazolidinylurea, imidazolidinyl urea, quaternium 15, polyaminopropyl biguanide. This antibacterial composition can be added to any kind of products such as body care and household products.

EP 1 216 691 A1

Printed by Jouve, 75001 PARIS (FR)

## Description

The present invention relates to an antibacterial composition comprising a synergistic mixture of a fragrance aldehyde and a preservative.

For decades several essential oils and fragrances derived from them have been known to exert antibacterial effect. In deodorant products, the natural essential oil ingredients like farnesol or $\alpha$-bisabolol are used to prevent the growth of malodor forming skin bacteria, especially *Corynebacteria.* US-A 4,966,754 discloses an essential oil mixture which is used to preserve cosmetic compositions. Fragrances which preserve skin lotions are disclosed in Seifen, Oele, Fette, Wachse; 1990, 116(9):357-359. In Seifen, Oele, Fette, Wachse; 1990, 122(11):789-793 various cosmetic products are disclosed which are solely preserved by fragrances which have an antibacterial effect. In some cases the concentration of such fragrances used for sufficient preservative effect is high. US-A 4,966,754 discloses a product comprising at least 2% of such a fragrance composition.

Among the antibacterial fragrance materials, long chain alcohols, either branched or unbranched, aromatic alcohols, phenols and aldehydes, both aromatic or aliphatic are most common. Said aromatic aldehydes have a particularly strong antibacterial activity. One such aldehyde is phenylacetaldehyde, a fragrance material occuring in nature especially in oils and headspace samples obtained from flowers, e.g. Lindenblossom or jasmine. It is used widely in the fragrance industry mainly for creating floral-green notes. A closely related synthetic analog of this material is 4-methylphenylacetaldehyde. It is used in perfumery for similar purposes as phenylacetaldehyde. It is commonly referred to as Syringa Aldehyde. These two aldehydes are normally used from traces up to 3% in fragrance oils. Besides these two materials there is a range of other aromatic aldehydes used in perfumery, e.g. benzaldehyde, cinnamic aldehyde or 3-phenyl-propionic aldehyde.

Preservatives are widely used in all kind of products to prevent them from microbial deterioration. Among the 10 most used preservatives in cosmetic formulations, Steinberg (Cosmetic and Toiletries ingredient Resource Series: Preservatives for Cosmetics, Allured publishing, 1996) lists Methyl-, Ethyl-, Propyl- and Butylparaben (Esters of parahydroxybenzoic acid). Other preservatives included in this short list are Imidazolidinyl Urea (sold under the trade name Germall 115 by ISP/Sutton), Diazolidinyl Urea (sold under the trade name Germall II by ISP/Sutton) and Quaternium-15 (1-(3-chloroallyl)-3,5,7 triaza-1-azoniaadamantane chloride; sold under the trade name Dowicil 200 by Dow Chemicals Company). Yet another preservative is Polyaminopropyl Biguanide (sold under the trade name Cosmocil CQ by Zeneca Biocides).

EP-A 0 570 794 discloses a synergistic combination of organic acids and perfumes as a new preservative system. J. Essent. Oil Res., 10, 293-297 May/Jun 1998 discloses the oil of fennel to act synergistically with parabens, which are common preservatives. Another example of a synergistic combination between essential oils and antibacterial agents is disclosed in EP-A 0 244 363.

Therein, a mixture of essential oils combined with bis-biguanido hexanes is described to have a synergistic effect against bacteria causing dental caries.

Such synergistic effects between essential oils and fragrance materials are rather the exception than the rule. It is generally accepted, that compounds which act on the same target in the cells, give in most cases only additive effects when used in combination. Since many common preservatives and fragrance materials are believed to be membrane disrupting agents, no person skilled in the art would predict synergism for combinations of preservatives with fragrance materials. In Cosmetics and Toiletries, 92(3):54-56, 1977 no synergistic effect was described when combining the antibacterial fragrance alcohol 2-phenylethanol with the membrane disrupting antimicrobials m-cresol or with benzalkonium chloride. A synergistic interaction was found when combining said fragrance material with organomercurial compounds, which are believed to act mainly on intracellular enzymes.

A synergistic combination of a preservative with a fragrance is especially interesting for the preservation of all types of perfumed products which need preservatives to be added to prevent them from microbial deterioration. Furthermore, similar synergistic combinations are also of interest when an antibacterial action is needed for any other reason. Thus for example cosmetic deodorants often contain fragrances and preservative compounds. In addition these products contain other antibacterial agents like Triclosan, which should prevent the growth of malodor forming microorganisms once the product has been applied to the skin. New compositions, where the synergistic effect of the preservatives and the fragrance gives enhanced antibacterial effect when applied to the skin, could make the use of such additional antibacterial agents obsolete.

It is an object of the present invention to provide an antibacterial composition comprising a synergistic mixture of a fragrance and a preservative.

By checkerboard titration analysis antibacterial fragrances belonging to different chemical classes have been tested in combination with the most common preservatives used in perfumed products. *Staphylococcus aureus* as gram positive and *Escherichia coli* as gram-negative standard organisms were used. Most of these tested combinations only give additive effect. However, it has been surprisingly found that a composition comprising a mixture of

(a) a fragrance aldehyde of the formula (I)

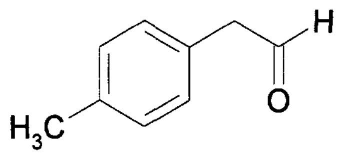

(I)

or of the formula (II)

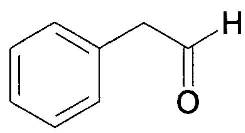

(II)

or a mixture thereof, and

(b) a preservative selected from the group of diazolidinyl urea, imidazolidinyl urea, quaternium 15, polyaminopropyl biguanide

has an outstanding synergistic effect against bacteria.

The combination of one of said fragrance aldehydes or a mixture thereof with Imidazolidinylurea, Diazolidinylurea and Quaternium-15 results in strong synergistic effects. A weaker synergism is observed if said fragrance aldehydes or a mixture thereof are combined with the preservative polyaminopropyl biguanide (Cosmocil CQ). This allows to reduce concentration of both, the fragrance aldehydes and the preservatives, significantly when they are used in combination to confer an antimicrobial effect to a product. Preferably a composition according to the present invention comprises 0.005 to 1% by weight of the final product of the fragrance aldehyde of formula I or II or a mixture thereof, and 0.005 to 0.5 % by weight of the preservative.

In a preferred embodiment of the present invention the fragrance aldehyde of the formula I or II or a mixture thereof is added together with further fragrance components to a mixture of the other ingredients. However, the fragrance aldehyde do not need to be added to the perfume, but can also be added directly as an active ingredient to the final product.

This antibacterial composition may contain additional ingredients, and can be added to any kind of finished products.

Such products can be any body care products which include all types of products which are applied in any manner directly to a person for cleansing or embellishment purposes. Such body care products include among others facial care products, deodorant and/or antiperspirant products, such as creams and lotions, such as cleansing creams, facial creams, shaving creams, hand lotions and balms, antipersperant and deodorant lotions and creams, cleansing gels, bath oils and gels, hair dressing gels, and creams and conditioners, make-up, suntan oils, lotions and creams and other cosmetic applications.

Furthermore such products can be any household product which needs preservative agents to be added. These household products can be cleaning products, laundry care products, surface care products and other products used for similar purposes.

The composition according to the present invention with this synergistic effect against bacteria can be used to preserve a product from microbiological deterioriation. In another application the combination according to the present invention is used to confer an enhanced antibacterial effect to cosmetic deodorant products, in order to prevent the growth of malodor forming *Corynebacteria* once these products are applied to the skin.

**Examples**

The synergistic action between individual perfume ingredients or perfume compositions on the one hand and antibacterial agents on the other hand has been determined by checkerboard titration. This method has been utilized in all examples and it is thus briefly described before the results of the different examples are described in detail.

Checkerboard titration is used to assess the antibacterial activity of combinations. The 'checkerboard' is the inhibitory pattern found for multiple combinations of two antimicrobial agents in concentrations equal to, above, and below their minimal inhibitory concentration for the organism being tested. These multiple combinations of antimicrobial agents are prepared in microtiter plates. The checkerboard consists then of columns which contain the same amount of one antimicrobial which is diluted along the x-axis and rows which each contain the same amount of the other antimicrobial (diluted along the y-axis). Thus each well of the microtiter plate is a unique combination of the two agents.
A method of quantitating synergy is the 'fractional inhibitory concentration' (FIC) index. For each row, the FIC index is calculated from the lowest concentration of agents necessary to inhibit growth. The FIC of each agent is derived by dividing the concentration of the agent present in that well of the microtiter plate by the minimal inhibitory concentration (MIC) needed of that agent alone to inhibit the organism. The FIC index is then the sum of these values for both antibacterial agents in that well of the microtiter plate. Fractional inhibitory concentration indices are then used as measure of synergy. When the lowest FIC index obtained in a microtiter plate in this way is equal to or less than 0.5, the combination is clearly synergistic. When the FIC index is around one, the combination is merely additive. A FIC index of two indicates indifference, and values above 2 indicate antagonism between the two compounds.
The FIC index is calculated for each row as follows:

$$FICindex = \frac{MICa}{MICA} + \frac{MICb}{MICB}$$

Where *MICa*, *MICb* are the minimal inhibitory concentration (MIC) of compound A and B in the mixture, and *MICA, MICB* are the MIC of A and B when used alone. The FIC indices indicated in the following tables show the lowest value obtained by evaluating each single row according to the above formula. Thus a FIC index of e.g. 0.375 indicates that one compound at 1/8 its MIC combined with ¼ of the MIC of the other compound gives inhibition of bacterial growth.
Besides the FIC index, in some examples the Fractional bacteriocidal concentration FBC index is determined. This value is obtained in the same way, with the exception that the minimal bacteriocidal concentrations (MBC) are determined for the compounds and their mixtures.
All examples were made using routine techniques for MIC determination. Briefly, bacteria were swabbed from agar plates and suspended in 4 ml of Müller-Hinton broth amended with 100 mg of Tween80 per Litre (MH-Tween) and incubated at 36° C for 16 hours. Following incubation the bacterial suspensions were diluted in MH-Tween to obtain a final cell density of $10^7$ colony forming units per ml. To each well of the microtiter plates 100 µl of such a diluted suspension of a different test organism was distributed. Stock solutions of the fragrance materials were prepared by dispersing the material by ultrasonication in MH-Tween broth. Stock solutions of the preservatives were prepared in MH-Tween. 100 µl per well of the conventional preservative was then added to the first row of the microtiter plates already containing the target organisms. Serial dilution series were then prepared starting at row A and continuing until row G, each time removing 100 µl from a well and transferring it to the next well. Row H received no conventional preservative, and was thus used to determine the MIC of the fragrance alone. 100 µl per well of the fragrance material was then added to the first column of the microtiter plate, and dilutions were prepared starting at column 1 and continuing to column 11. Column 12 received no fragrance material, and was thus used to determine the MIC of the preservative alone. To determine the bacteriocidal concentrations, aliquots (about 0.5 µl) of each well were transferred at certain time intervals to new microtiter plates containing 100µl per well of MH-Tween without antibacterial agents, and these replicate plates were checked for surviving microorganisms by measuring growth after 24 h. The plates were covered with plastic films and incubated for 24 h at 36° C with shaking at 250 rpm. After 24 h, 50 µl of a solution containing 0.4% Glucose and 0.015% of Tetrazolium violet was added, and the red dye was measured with a microplate reader to determine bacterial growth. As test organisms *Escherichia coli* ATCC 10536, *S. aureus* ATCC 6538 and *Corynebacterium* group G strain Ax7, a representative of the human malodor forming axilla flora, were used.

**Example 1: Synergistic bacteriostatic action of aromatic fragrance aldehydes and biocides**

MIC and FIC index values for aromatic fragrance aldehydes were determined by the method described above. Results are average values of 2-4 experiments and are listed in Table 1. It is evident from this example, that only combinations with Phenylacetaldehyde and Syringa aldehyde give synergistic action with the preservatives. Indeed, this specific synergistic action is quite strong, especially for *Escherichia coli* and *Corynebacterium.* Thus in most cases ¼ of the MIC of the fragrance combined with ¼ of the MIC of the biocides or even less gives sufficient inhibition. This level is for *E. coli* e.g. 0.025% of Phenylacetaldehyde combined with 0.0156% of Imidazolidinylurea.
FIC index values as low as 0.25 were observed for Corynebacterium group G, indicating that 1/8 the concentration of each compound is necessary, compared to the level when they are used individually, or expressed in concentrations Phenylacetaldehyde can be used at a concentration of 0.00195% when it is combined with 0.0156% Imidazolidinylurea.

Table 1:

| Growth inhibitory synergistic action of aldehydes and biocides | | | |
|---|---|---|---|
| **Organism** | **E. coli** | **S. aureus** | **Coryne-bacterium group G** |
| MIC Imidazolidinylurea (IU) | 0.063 | 0.063 | 0.063 |
| MIC Quaternium 15 (Q15) | 0.063 | 0.016 | 0.016 |
| MIC Diazolidinylurea (DU) | 0.031 | 0.031 | n.d |
| MIC Cosmocil CQ (CQ) | 0.0006 | 0.0006 | n.d |
| MIC Phenylacetaldehyde (PAA) | 0.097 | 0.027 | 0.031 |
| MIC Syringa Aldehyde (SA) | 0.063 | 0.039 | 0.016 |
| MIC Cinnamic aldehyde (CA) | 0.031 | 0.031 | n.d. |
| MIC Benzaldehyde (BA) | 0.125 | 0.25 | n.d. |
| MIC 3-Phenylpropionicaldehyde (PPA) | 0.031 | 0.125 | n.d. |
| **FIC index IU+PAA** | **0.406** | **0.563** | **0.250** |
| **FIC index IU+SA** | **0.594** | **0.469** | **0.375** |
| FIC index IU+CA | 2 | 1 | n.d. |
| FIC index IU+BA | 1 | 1 | n.d. |
| FIC index IU+PPA | 1 | 1 | n.d. |
| **FIC index Q15+PAA** | **0.281** | **0.625** | **0.313** |
| **FIC index Q15+SA** | **0.563** | **0.500** | **0.500** |
| FIC index Q15+CA | 0.750 | 1 | n.d. |
| FIC index Q15+BA | 1 | 1 | n.d. |
| FIC index Q15+PPA | 1 | 1 | n.d. |
| **FIC index DU+PAA** | **0.250** | **0.500** | n.d |
| **FIC index DU+SA** | **0.500** | **0.375** | n.d |
| **FIC index CQ+PAA** | **0.500** | **0.500** | n.d |
| **FIC index CQ+SA** | 0.625 | 0.750 | **n.d** |

**Example 2: Synergistic bacteriocidal action of aromatic fragrance aldehydes and biocides**

In another set of experiments the FBC instead of the FIC values were determined for similar combinations. Similar synergistic interactions were found. They were particularly strong for *E. coli* when measured after 6 hours, when all FBC values were around 0.25. The slow bacteriocidal action measured in these experiments is necessary for compounds or mixtures which are used as preservatives, since for product preservation not only the growth of bacteria in the product must be stopped, but contaminating organisms should also be killed once the enter a product during manufacturing or during use.

Table 2.

| Slow bacteriocidal effect of combinations of aldehydes and biocides | | | |
|---|---|---|---|
| | **E. coli/5h** | **E. coli /24h** | **S. aureus/24h** |
| MBC Imidazolidinylurea (IU) | 0.500 | 0.095 | 0.125 |
| MBC Quaternium 15 (Q15) | 0.313 | 0.125 | 0.031 |
| MBC Diazolidinylurea (DU) | n.d. | 0.031 | 0.063 |
| MBC Cosmocil CQ (CQ) | n.d. | 0.0006 | 0.0006 |
| MBC Phenylacetaldehyde (PPA)) | 0.052 | 0.031 | 0.016 |
| MBC Syringa Aldehyde (SA) | 0.046 | 0.063 | 0.031 |
| MBC Cinnamic aldehyde (CA) | n.d. | 0.063 | 0.063 |
| MBC Benzaldehyde (BA) | n.d. | 0.5 | 0.5 |
| MBC 3-Phenylpropionicaldehyde (PPA) | n.d. | 0.063 | 0.125 |
| **FBC index IU+PPA** | **0.229** | **0.500** | **0.625** |
| **FBC index IU+SA** | **0.271** | **0.375** | **0.500** |

Table 2. (continued)

| Slow bacteriocidal effect of combinations of aldehydes and biocides | | | |
|---|---|---|---|
| | **E. coli/5h** | **E. coli /24h** | **S. aureus/24h** |
| FBC index IU+CA | n.d. | 0.750 | 2 |
| FBC index IU+BA | n.d. | 1 | 2 |
| FBC index IU+PPA | n.d. | 0.75 | 1 |
| **FBC index Q15+PPA** | **0.245** | **0.250** | **0.625** |
| **FBC index Q15 +SA** | **0.318** | **0.313** | **0.500** |
| FBC index Q15+CA | n.d. | 0.750 | 1 |
| FBC index Q15+BA | n.d. | 1 | 2 |
| FBC index Q15+PPA | n.d. | 1 | 2 |
| **FBC index DU+PAA** | n.d. | **0.250** | **0.625** |
| FBC index DU+SA | n.d. | **0.500** | **0.375** |
| **FBC index CQ+PAA** | **n.d.** | **0.500** | **0.625** |
| FBC index CQ+SA | **n.d.** | **0.625** | **1** |

**Example 3: Synergistic bacteriostatic action of a Fragrance composition with and without aromatic fragrance aldehydes added.**

In a further set of experiments it was determined whether the inventive combinations give the desired effect also when the fragrance aldehydes are part of a complex perfume oil instead of being used individually. Perfume oils with and without aromatic fragrance aldehyde added were prepared as described in Table 3. MIC and FIC index values of these oils were determined in combinations with the preservatives as described above. The results from Table 4 clearly show, that a strong synergistic action (with FIC index values of 0.234 and 0.340) is obtained with a perfume oil containing 3% of Phenylacetaldehyde, but that this effect is lost when said compound is replaced by a solvent like Dipropyleneglycol. Indeed, the effective concentration of Phenylacetaldehyde giving the bacteriostatic concentrations in combination with the preservatives is at least as low or even lower as in Example 1. Thus, the perfume has bacteriostatic activity only when used at 2%, on the other hand ¼ MIC of Quaternium 15 (=0.0156%) can be combined with 0.125% of fragrance to give inhibition of growth of *E. coli*, thus the effective concentration of Phenylacetaldehyde in this combination is 0.00375%.

Table 3.

| Composition of perfumes used in example 3 | | |
|---|---|---|
| | Perfume A | Perfume B |
| Benzylacetate | 40 | 40 |
| Citronellylacetate | 35 | 35 |
| cis-3-hexenylacetate | 5 | 5 |
| Hexylacetate | 153 | 153 |
| Prenylacetate | 5 | 5 |
| Agrumex | 80 | 80 |
| α-hexyl-cinnamicaldehyde | 50 | 50 |
| Phenylacetaldehyde 85% in 2-Phenylethylalcohol | 30 | - |
| Dipropyleneglycol | - | 30 |
| Ambrettolide | 20 | 20 |
| Allyl-cyclohexylacetate | 10 | 10 |
| Ethyle-decadienoate | 1 | 1 |
| Galaxolide at 50% in BB | 40 | 40 |
| Givescone | 15 | 15 |

Table 3. (continued)

| Composition of perfumes used in example 3 | | |
|---|---|---|
| | Perfume A | Perfume B |
| Isobutyrate phenoxyethyle | 210 | 210 |
| Lilial | 60 | 60 |
| Nectaryl | 30 | 30 |
| Verdylpropionate | 150 | 150 |
| Hexylsalicylate | 60 | 60 |
| Veloutone | 6 | 6 |
| Total | 1000 | 1000 |

Table 4.

| The synergistic action of common preservatives in combination with perfume oils with and without Phenylacetaldehyde added | |
|---|---|
| | ***E. coli*** |
| MIC perfume A | 2% |
| MIC perfume B | >2% |
| FIC index of perfume A combined with Imidazolidinylurea | 0.340 |
| FIC index of perfume A combined with Quaternium 15 | 0.234 |
| FIC index of perfume B combined with Imidazolidinylurea | >= 2 |
| FIC index of perfume B combined with Quaternium 15 | >= 2 |

## Claims

**1.** An antibacterial composition comprising a synergistic mixture of

(a) a fragrance aldehyde of the formula (I)

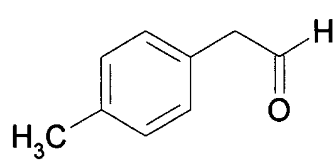

(I)

or of the formula (II)

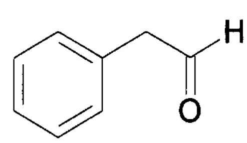

(II)

or a mixture thereof, and

(b) a preservative selected from the group of diazolidinyl urea, imidazolidinyl urea, quaternium 15, and polyaminopropyl biguanide.

2. Composition according to any of the preceding claims comprising 0.005 to 0.1% by weight of the fragrance aldehyde of formula I or II or a mixture thereof.

3. Composition according to any of the preceding claims comprising of 0.005 to 0.5% by weight of the preservative.

4. A consumer product comprising a composition according to any of the preceding claims.

5. A body care product comprising a composition according to claims 1 to 4.

6. A facial care product comprising a composition according to claims 1 to 4.

7. A deodorant and/or an antiperspirant product comprising a composition according to claims 1 to 4.

8. A household product comprising a composition according to claims 1 to 4.

9. Method for preparing a product according to claims 5 to 9 by adding the fragrance aldehyde of formula I or II or a mixture thereof together with further fragrance components to a mixture of the other ingredients.

10. Method for preparing a product according to claim 5 to 9 by adding the fragrance aldehyde of formula I or II or a mixture thereof separately from further fragrance components to the other ingredients.

European Patent Office

# EUROPEAN SEARCH REPORT

Application Number
EP 00 12 8472

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,A | EP 0 570 794 A (GIVAUDAN ROURE INT) 24 November 1993 (1993-11-24) * the whole document * --- | 1-10 | A61K7/46 A61K7/32 A61K7/48 C11D3/00 C11D3/28 C11D3/32 |
| D,A | EP 0 244 363 A (WARNER LAMBERT CO) 4 November 1987 (1987-11-04) * the whole document * --- | 1-10 | |
| A | US 4 495 079 A (GOOD ALLEN H) 22 January 1985 (1985-01-22) * column 4, line 26 - line 48 * --- | 1-10 | |
| A | EP 0 451 889 A (UNILEVER PLC ;UNILEVER NV (NL)) 16 October 1991 (1991-10-16) * the whole document * --- | 1-10 | |
| A | EP 0 980 863 A (GIVAUDAN ROURE INT) 23 February 2000 (2000-02-23) * examples * ----- | 1-10 | |

TECHNICAL FIELDS SEARCHED (Int.Cl.7)
A61K
C11B
C11D

The present search report has been drawn up for all claims

| Place of search | Date of completion of the search | Examiner |
|---|---|---|
| MUNICH | 13 June 2001 | Pregetter, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 00 12 8472

This annex lists the patent family members relating to the patent documents cited in the above-mentioned European search report.
The members are as contained in the European Patent Office EDP file on
The European Patent Office is in no way liable for these particulars which are merely given for the purpose of information.

13-06-2001

| Patent document cited in search report | Publication date | Patent family member(s) | Publication date |
|---|---|---|---|
| EP 0570794 A | 24-11-1993 | JP 6279208 A | 04-10-1994 |
| EP 0244363 A | 04-11-1987 | AU 582047 B | 09-03-1989 |
| | | AU 7187887 A | 05-11-1987 |
| | | DK 223387 A | 03-11-1987 |
| | | FI 871918 A | 03-11-1987 |
| | | JP 62289511 A | 16-12-1987 |
| | | ZA 8702857 A | 25-11-1987 |
| US 4495079 A | 22-01-1985 | NONE | |
| EP 0451889 A | 16-10-1991 | AT 114962 T | 15-12-1994 |
| | | AU 640205 B | 19-08-1993 |
| | | AU 7298391 A | 26-09-1991 |
| | | BR 9101077 A | 05-11-1991 |
| | | CA 2038382 A,C | 21-09-1991 |
| | | DE 69105586 D | 19-01-1995 |
| | | DE 69105586 T | 20-04-1995 |
| | | US 5306707 A | 26-04-1994 |
| EP 0980863 A | 23-02-2000 | AU 4453399 A | 09-03-2000 |
| | | BR 9903629 A | 26-09-2000 |
| | | JP 2000109457 A | 18-04-2000 |

EPO FORM P0459

For more details about this annex : see Official Journal of the European Patent Office, No. 12/82